# EUROPÄISCHE PATENTANMELDUNG

(11) **EP 0 692 274 A2**
(43) Veröffentlichungstag der Anmeldung: **17.01.1996**
(21) Anmeldenummer: 95110683.0
(22) Anmeldetag: 08.07.1995
(51) Int. Cl.: A61M 16/06

(54) **Individualmaske**

(30) Priorität: 15.07.1994 DE 9411495 U
(71) Anmelder: GOTTLIEB WEINMANN GERÄTE FÜR MEDIZIN UND ARBEITSSCHUTZ GMBH & CO., D-22525 Hamburg (DE)
(72) Erfinder: Ebers, Manfred, D-22457 Hamburg (DE)
(74) Vertreter: Liebelt, Rolf, Dipl.-Ing.

(57) **Zusammenfassung**

Eine Individualmaske zur künstlichen Beatmung eines Patienten weist einen mindestens dessen Nase im Bereich der Nasenlöcher überspannenden Maskenkörper 4 auf. Der Maskenkörper 4, an den mindestens eine Gaszuführung anschließbar ist und an dem Haltebänder anordenbar sind, liegt am Gesicht des Patienten mit einer als Hohlkörper 4, der eine an die Kontur des Gesichtes des Patienten anpaßbare, federnd nachgebende Füllung aufweist, ausgebildeten Wulst 2 an. An der Wulst 2 ist eine dünne elastische Haut 1 gehalten, die den zum Gesicht des Patienten weisenden Bereich des Maskenkörpers 4 überspannt und eine Öffnung 6 zum Einführen der Nase des Patienten in den vom Maskenkörper 4 umschlossenen Raum aufweist.

## Beschreibung

Die Erfindung betrifft eine Individualmaske zur künstlichen Beatmung eines Patienten mit einem mindestens dessen Nase im Bereich der Nasenlöcher überspannenden sowie an dessen Gesicht mit einer als Hohlkörper, der eine an die Kontur des Gesichtes des Patienten anpaßbare, federnd nachgebende Füllung aufweist, ausgebildeten Wulst anliegenden Maskenkörper, an den mindestens eine Gaszuführung anschließbar ist und an dem Haltebänder anordenbar sind.

Bei derartigen aus der DE-PS 4 212 259 bekannten als Massenprodukt vorgefertigten Masken wird zum Anpassen der als Dichtung zwischen Gesicht und Maske dienenden Wulst, deren Hohlkörper mit einem flüssigen oder pastösen Kunststoff, der formstabil und federnd nachgebend aushärtet, gefüllt und danach die Maske auf das Gesicht aufgelegt, wobei der noch weiche Kunststoff und damit die Dichtfläche der Wulst die Kontur des Gesichtes annehmen. Trotz dieser individuellen Anpassung der Dichtfläche an die Kontur des Gesichtes des Patienten wird eine vollständige Abdichtung zwischen Maskenrand und Gesicht nur dann erhalten, wenn die Atemmaske mit einem gewissen Druck an das Gesicht gepreßt wird, was bei häufiger und lang andauernder Benutzung einer Atemmaske z. B. bei der Behandlung einer Schlafapnoe als lästig empfunden wird. Wenn auch geringe Undichtigkeiten die Wirksamkeit der künstlichen Beatmung nicht zu beeinträchtigen vermögen, so können die entweichenden Gase Folgeerkrankungen wie Bindehautentzündungen bewirken oder auch nur als ständiger Luftstrom störend sein.

Aufgabe der Erfindung ist es nun, bei gleichzeitiger Verbesserung des Tragekomforts eine sichere Abdichtung zwischen dem Gesicht eines Patienten und einer Atemmaske zu schaffen.

Diese Aufgabe wird erfindungsgemäß ausgehend von einer Atemmaske der eingangs beschriebenen Gattung dadurch gelöst, daß an der Wulst eine dünne elastische Haut gehalten ist, die den zum Gesicht des Patienten weisenden Bereich des Maskenkörpers überspannt und eine Öffnung als Zugang für die Nase des Patienten zu dem vom Maskenkörper umschlossenen Raum aufweist.

Die neuerungsgememäß den zum Gesicht des Patienten weisenden Bereich des Maskenkörpers überspannende Haut wird bei angelegter Maske vom Druck des Atemgases gegen das Gesicht und den Nasenrücken des Patienten gedrückt. Auf diese Weise wird nach der Erfindung eine großflächig wirkende Dichtung erhalten, so daß wegen der optimalen Sitz- oder Paßform der Maske, die durch die individuelle Anpassung der Wulst an die Kontur des Gesichtes des Patienten erhalten wird, mit den Haltebändern die Maske nicht an das Gesicht gepreßt, sondern nur noch am Gesicht anliegend gehalten werden muß. Dieser durch die Verringerung des Anpreßdruckes der Maske an das Gesicht des Patienten erzielte Tragekomfort kann bei einer zweckmäßigen Weiterbildung der Erfindung noch dadurch verbessert werden, daß die Haut aus einem atmungsaktiven und wasserdampfdurchlässigen Werkstoff wie ein synthetisches Block-Co-Polymer und/oder mit unebener Oberfläche besteht, wodurch ohne Beeinträchtigung der Dichtfunktion ein Ankleben der Haut auf dem Gesicht des Patienten verhindert und eine Mikroflüssigkeitszirkulation zwischen Haut und Gesicht ermöglicht wird.

Ein Ausführungsbeispiel der Erfindung wird noch an Hand der Zeichnungen beschrieben. Es stellen dar:

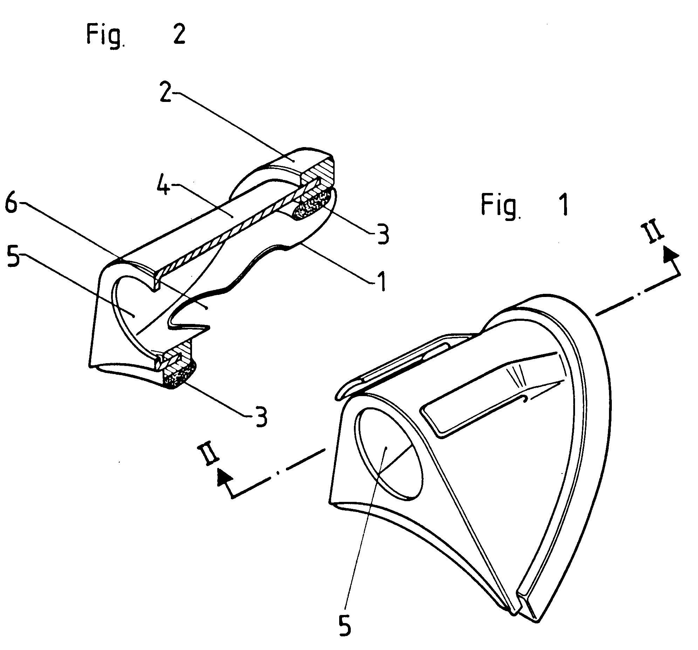

- Fig. 1: eine schematisch perspektivische Ansicht einer Atemmaske,
- Fig. 2: eine Schnittansicht längs der Linie II - II der Fig. 1.

Die mit nicht gezeigten Haltebändern am Kopf eines Patienten befestigbare Atemmaske nach der Erfindung besteht aus einem Maskenkörper 4 mit einer Bohrung 5 zum Anschluß einer Atemgasleitung (nicht dargestellt). Am freien Rand des Maskenkörprs 4 ist eine Wulst 2 mit integriertem Hohlkörper 3 aus einem elastischen Werkstoff, vorzugsweise Gummi, befestigt. Der Hohlkörper 3 dieser Wulst 2, die auf dem Gesicht des Patienten (nicht dargestellt) aufliegt, ist mit einer Füllung aus einem im ausgehärteten Zustand formstabilen sowie federnd nachgebenden Kunststoff versehen. An der Wulst 2 ist eine dünne elastische Haut 1 gehalten, die den zum Gesicht des Patienten weisenden Bereich des Maskenkörpers 4 überspannt und eine Öffnung 6 zum Einführen der Nase des Patienten in den vom Maskenkörper 4 umschlossenen Raum aufweist.

## Patentansprüche

1. Individualmaske zur künstlichen Beatmung eines Patienten mit einem mindestens dessen Nase im Bereich der Nasenlöcher überspannenden sowie an dessen Gesicht mit einer als Hohlkörper, der eine an die Kontur des Gesichtes des Patienten anpaßbare, federnd nachgebende Füllung aufweist, ausgebildeten Wulst anliegenden Maskenkörper, an den mindestens eine Gaszuführung anschließbar ist und an dem Haltebänder anordenbar sind, dadurch gekennzeichnet, daß an der Wulst (2) eine dünne elastische Haut (1) gehalten ist, die den zum Gesicht des Patienten weisenden Bereich des Maskenkörpers (4) überspannt und eine Öffnung (6) zum Einführen der Nase des Patienten in den vom Maskenkörper (4) umschlossenen Raum aufweist.

2. Individualmaske nach Anspruch 1, dadurch gekennzeichnet, daß die Haut (1) aus einem atmungsaktiven und wasserdampfdurchlässigen Werkstoff besteht.

3. Individualmaske nach Anspruch 1 oder 2, dadurch gekennzeichnet, daß die Haut (1) aus einem Werkstoff mit unebener Oberfläche besteht.
